# EUROPÄISCHE PATENTSCHRIFT

(11) **EP 1 078 906 B1**
(45) Veröffentlichungstag und Bekanntmachung des Hinweises auf die Patenterteilung: **08.12.2004**
(21) Anmeldenummer: 00114218.1
(22) Anmeldetag: 03.07.2000
(51) Int. Cl.: C07C 29/76, C07C 31/22, B01D 61/16, C02F 1/44

(54) **Verfahren zum Entfernen von kurzkettigen Fettsäuren aus einer wässrigen Glycerinlösung**
Process for the removal of short chain fatty acids from aqueous glycerol solutions
Procédé d'élimination des acides gras à chaîne courte présents dans une solution aqueuse de glycérol

(30) Priorität: 17.08.1999 DE 19938874
(43) Veröffentlichungstag der Anmeldung: 28.02.2001
(73) Patentinhaber: MG Technologies AG, 60325 Frankfurt am Main (DE); Peter Greven Fett-Chemie GmbH & Co.KG, 53902 Bad Münstereifel (DE)
(72) Erfinder: Boensch, Rudolf, Dr., 55299 Nackenheim (DE); Mitschke, Peter, 63477 Maintal (DE); Buchold, Henning, Dr., 63452 Hanau (DE); Greven, Heinzpeter, 65760 Eschborn (DE); Dederichs, Hans-Theo, 53947 Nettersheim (DE); Grossgarten, Thomas, 53507 Dernau (DE)
(74) Vertreter: Revesz, Veronika

(56) Entgegenhaltungen:
- EP-A- 0 358 255
- DE-A- 4 325 786
- DATABASE WPI Section Ch, Week 198643 Derwent Publications Ltd., London, GB; Class D17, AN 1986-283915 XP002150343 & SU 1 216 176 A (SYNTH RUBBER RES), 7. März 1986 (1986-03-07)
- "Ullmann's Encyclopedia of Industrial Chemistry, 5. Aufl., Bd. A 12, Seiten 480-483" 1986 , VERLAGSGESELLSCHAFT CHEMIE , WEINHEIM XP002150341 * Seite 481, Punkt 3.1.3 *
- "Kirk-Othmer, Encyclopedia of Chemical Technology, 3. Aufl., Bd. 11, Seiten 924-26" 1980 , JOHN WILEY & SONS , NEW YORK XP002150342 * Seite 925, Zeile 28 - Seite 926, Zeile 12 *
- DATABASE WPI Section Ch, Week 198336 Derwent Publications Ltd., London, GB; Class A60, AN 1983-755485 XP002150344 & JP 58 126827 A (NIPPON OILS & FATS CO LTD), 28. Juli 1983 (1983-07-28)

## Beschreibung

Die Erfindung betrifft ein Verfahren zum Entfernen von kurzkettigen Fettsäuren mit 4 bis 10 C-Atomen pro Molekül aus einer Fettsäuren enthaltenden wäßrigen Glycerinlösung. Die zu behandelnde wäßrige Glycerinlösung stammt zum Beispiel aus der Spaltung von Fetten oder Ölen mit Wasser, wobei man ein 5 bis 30 Gew.% Glycerin enthaltendes Spaltwasser erhält. Ein weiteres Verfahren, bei welchem eine wäßrige Glycerinlösung anfällt, welche kurzkettige Fettsäuren enthält, ist die Neutralölverseifung, wobei Unterlaugen-Glycerin gebildet wird.

In bekannter Weise wird eine solche Glycerinlösung z.B. durch Zugabe anorganischer Säuren und Fällungsmittel behandelt.

Die DE-A-4325786 beschreibt ein Verfahren zur Entfernung störender Substanzen aus einem Glycerin-Wasser-Gemisch. Diese Substanzen werden in die Hochdruckspaltung zurückgeführt, wobei ein,Plattenphasentrenner als Reinigungsaggregat vor der Mikrofiltration eingesetzt wird. Kurzkettige Fettsäuren werden bei diesem Verfahren nicht als zweites Produkt gewonnene.

Die SU 1216176 beschreibt die Trennung von Fettsäuren von Glycerin-Wasser-Gemischen, wobei das Glycerin-Wasser-Gemisch anschließend hydriert und gefiltert wird. Die EP-A-358255 beschreibt Einzelheiten der Reinigung von rohem Glycerin durch Mikrofiltration und Ultrafiltration. Weitere Beschreibungen zu Verfahren bei der Trennung von Fettsäuren von Glycerin-Wasser-Gemischen finden sich in den Schriften JP58126827, Ullmann's Encyclopedia of Industrial Chemistry (5. Auflage, Bd. A 12, S. 480-483) und Kirk-Othmer, Encyclopedia of Chemical Technology (3. Auflage, Bd. 11, S. 924-926)

Der Erfindung liegt die Aufgabe zugrunde, beim eingangs genannten Verfahren die kurzkettigen Fettsäuren kostengünstig und möglichst vollständig abzutrennen, um reines Glycerin gewinnen zu können. Erfindungsgemäß geschieht dies dadurch, daß man die wäßrige Glycerinlösung aus einer als Zentrifuge ausgebildeten Grobreinigung in den Pumpenvorlagenbehälter. leitet und im Pumpenvorlagenbehälter die Löslichkeitsgrenze für kurzkettige Fettsäuren im Wasser überschreitet, wobei sich kurzkettige Fettsäuren vom Wasser trennen und sich als geschlossene Schicht auf der wässrigen Glycerinlösung sammeln und dass die kurzkettigen Fettsäuren aus dem Pumpenvorlagenbehälter abgezogen und aus dem Verfahren entfernt werden.

Die im Pumpenvorlagenbehälter abgetrennten Fettsäuren kann man als Produkt gewinnen, und sie werden nicht, wie das aber bei ähnlichen, bekannten Verfahren der Fall ist, stets verworfen und zum Beispiel durch Verbrennen entsorgt. Die als Permeat gewonnene wäßrige Glycerinlösung kann mit wenig Aufwand bis zu Glycerin in Pharmaqualität weiter behandelt werden. Dadurch, daß die kurzkettigen Fettsäuren entfernt wurden, wird das Verseifungsäquivalent im Glycerin abgesenkt und die Hitzestabilität des Pharma-Glycerins erhöht.

Die Filtrationseinheit, welche dem Pumpenvorlagenbehälter nachgeschaltet ist, kann in verschiedener Weise ausgestaltet sein. Vorzugsweise arbeitet man hier mit der an sich bekannten Mikrofiltration, bei welcher man geeignete Membranen mit Porenweiten üblicherweise im Bereich von 0,02 bis 10 µm einsetzt. Dabei trennt man einerseits Partikel aus der wäßrigen Lösung ab und andererseits werden Emulsionen gebrochen, wenn man mit der Überströmungstechnik (cross flow) arbeitet. Falls man Filtrationselemente aus Polypropylen verwendet, leitet man die Glycerinlösung mit Temperaturen im Bereich von 30 bis 90°C in die Mikrofiltration.

Anstelle der Mikrofiltration kann man auch mit der Ultrafiltration arbeiten, wobei im Unterschied zur Mikrofiltration Membranen mit Porenweiten von 2000 bis 500000 Dalton verwendet werden. Eine weitere Möglichkeit bietet die Nanofiltration, bei welcher die Porenweiten der Membranen unterhalb von 5000 Dalton liegen.

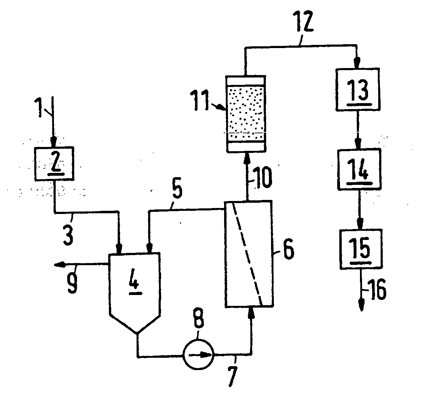

Ausgestaltungsmöglichkeiten der Verfahrensführung werden mit Hilfe der Zeichnung erläutert, welche ein Fließschema zeigt.

Die zu behandelnde wäßrige Glycerinlösung kommt aus der Leitung (1), sie enthält kurzkettige Fettsäuren mit 4 bis 10 C-Atomen pro Molekül und üblicherweise weitere Verunreinigungen, zum Beispiel Reste von Fetten, Seifen und Ölen. In einer Grobreinigung (2) mittels einer Zentrifuge, entfernt man grobe Schmutzstoffe und insbesondere auch flockige Seifen und ungelöste organische Verunreinigungen. Die grob gereinigte Lösung wird durch die Leitung (3) in einen Pumpenvorlagenbehälter (4) gegeben, dem man auch eine Fettsäuren enthaltende wäßrige Lösung aus der Leitung (5) zuführt. Bei dieser Lösung handelt es sich um das Retentat aus der Filtrationseinheit (6).

Aus dem unteren Bereich des Behälters (4) zieht man durch die Leitung (7) unter der Wirkung der Pumpe (8) ständig Flüssigkeit ab und führt sie durch die Filtrationseinheit (6). Das aus der Einheit (6) abgezogene Permeat strömt durch die Leitung (10) in einen Anionenaustauscher (11), in welchem restliche Fettsäuren gebunden und somit aus der Glycerinlösung entfernt werden. Bei Bedarf kann sich an den Anionenaustauscher ein Kationenaustauscher zur weiteren Reinigung und eine Entfärbung durch Aktivkohle anschließen.

Um Glycerin von hoher Reinheit und insbesondere auch von Pharmaqualität zu gewinnen, wird die aus dem Anionenaustauscher (11) kommende Lösung durch die Leitung (12) zunächst durch eine Eindampfung (13) und dann noch durch eine Destillation (14) und eine Bleichung (15) geführt. In der Bleichung verwendet man in an sich bekannter Weise zum Beispiel Aktivkohle. Das in der Leitung (16) anfallende Glycerin ist wasserfrei und zu Pharmazwecken geeignet.

Das durch die Leitung (5) aus der Filtrationseinheit (6) abgezogene Retentat erhöht im Vorlagenbehälter (4) den Anteil der kurzkettigen Fettsäuren, da sich die wäßrige Glycerinlösung zum weitaus größten Teil im Permeat der Leitung (10) wiederfindet. Nach einiger Betriebszeit, während der ständig Flüssigkeit durch die Leitungen (3) und (5) in den Behälter (4) fließt und Lösung durch die Leitung (7) in die Filtrationseinheit (6) eintritt, überschreitet die Konzentration der kurzkettigen Fettsäuren die Löslichkeitsgrenze im Behälter (4). Von diesem Betriebszeitpunkt ab, der für jede Anlage unterschiedlich ist, trennen sich die kurzkettigen Fettsäuren vom Wasser und schwimmen als geschlossene Schicht auf der Glycerinlösung im Behälter (4). Von nun an ist es ohne Schwierigkeiten möglich, die kurzkettigen Fettsäuren durch die Leitung (9) aus dem Behälter (4) abzuziehen. Bei den üblichen wäßrigen Glycerinlösungen, die durch die Leitung (3) herangeführt werden, handelt es sich bei kurzkettigen Fettsäuren der Leitung (9) um solche mit 4 bis 10 C-Atomen pro Molekül, z. B. Capronsäure, Capricsäure und Caprylsäure.

### Beispiel 1:

In einer der Zeichnung entsprechenden Anlage wird folgendermaßen gearbeitet: Aus einer Ölspaltung mit Wasser bei 250°C und 50 bar erhält man eine Wasserphase mit 20 Gew.-% Glycerin und einem Verseifungsäquivalent (gemäß British Standard No. 5711/21) von 2,5 Milliäquivalent pro 100 g Glycerin. In einer Zentrifuge (2) trennt man grobe Verunreinigungen ab, dabei bleibt das Verseifungsäquivalent ungeändert. Nach einer Anfahrphase fließen in den niveaugeregelten Behälter (4) zwei Ströme durch die Leitungen (3) und (5), wobei das Retentat der Leitung (5) 99 Gew.-% der kurzkettigen (C₄ bis C₁₀) Fettsäuren enthält, die in der Leitung (3) in den Behälter (4) eintreten. Durch die Leitung (9) werden die kurzkettigen Fettsäuren ausgeschleust, die Flüssigkeit in der Leitung (7) enthält noch 0,01 Gew.-% kurzkettige Fettsäuren, gelöst in Wasser.

Die Mikrofiltration (6) arbeitet nach dem Überströmungsprinzip mit handelsüblichen Keramikmembranen (von Firma Schumacher, Crailsheim, Deutschland). Die Temperatur in der Leitung (7) beträgt 70°C. Das Permeat in der Leitung (10) hat ein Verseifungsäquivalent von 1,1 Milliäquivalent pro 100 g Glycerin und enthält noch Reste von Farbstoffen, Geruchsstoffen und Partialglyceriden sowie anorganische Kationen und Anionen. In einem Kationenaustauscher (Lewatit von Bayer) und einem anschließenden Anionenaustauscher (11) (Lewatit) werden die Verunreinigungen nahezu vollständig entfernt. Das Glycerin der Leitung (16) hat Pharmaqualität gemäß British Standard.

**Beispiel 2:**

## Patentansprüche

1. Verfahren zum Entfernen von kurzkettigen Fettsäuren mit 4 bis 10 C-Atomen pro Molekül aus einer Fettsäure enthaltenden wässrigen Glycerinlösung, wobei man die wäßrige Glycerinlösung aus einer als Zentrifuge ausgebildeten Grobreinigung in den Pumpenvorlagenbehälter und anschließend in eine Filtrationseinheit leitet, aus welcher man Glycerinlösung als Permeat und eine kurzkettige Fettsäuren enthaltendes Retentat abzieht, wobei man das Retentat zurück in den Pumpenvorlagenbehälter leitet,
**dadurch gekennzeichnet,**
**dass** im Pumpenvorlagenbehälter die Löslichkeitsgrenze für kurzkettige Fettsäuren im Wasser überschritten wird, wobei sich kurzkettige Fettsäuren vom Wasser trennen und sich als geschlossene Schicht auf der wässrigen Glycerinlösung sammeln und dass die kurzkettigen Fettsäuren aus dem Pumpenvorlagenbehälter abgezogen und aus dem Verfahren entfernt werden und daß man das Permeat über einen Anionenaustauscher leitet und dabei die restlichen kurzkettige Fettsäuren aus der Glycerinlösung entfernt.

2. Verfahren nach Anspruch 1, **dadurch gekennzeichnet, daß** die Filtrationseinheit als Mikrofiltration ausgestaltet ist.

3. Verfahren nach Anspruch 1, **dadurch gekennzeichnet, daß** die Filtrationseinheit als Nanofiltration ausgestaltet ist.

4. Verfahren nach Anspruch 1, **dadurch gekennzeichnet, daß** die Filtrationseinheit als Ultrafiltrationseinheit ausgestaltet ist.

## Claims

1. A process for removing short-chain fatty acids having 4 to 10 C atoms per molecule from an aqueous glycerol solution containing fatty acid, wherein the aqueous glycerol solution is passed out of a coarse purification stage in the form of a centrifuge into the pump feed tank and then into a filtration unit, from which glycerol solution as permeate and a retentate containing short-chain fatty acids is withdrawn, the retentate being passed back into the pump feed tank,
**characterised in that**
in the pump feed tank the solubility limit for short-chain fatty acids in the water is exceeded, with short-chain fatty acids separating from the water and collecting as a closed layer on the aqueous glycerol solution, and that the short-chain fatty acids are withdrawn from the pump feed tank and removed from the process and
that the permeate is passed over an anion exchanger and in so doing the residual short-chain fatty acids are removed from the glycerol solution.

2. A process according to Claim 1, **characterised in that** the filtration unit is in the form of a microfiltration stage.

3. A process according to Claim 1, **characterised in that** the filtration unit is in the form of a nanofiltration stage.

4. A process according to Claim 1, **characterised in that** the filtration unit is in the form of an ultrafiltration stage.

## Revendications

1. Procédé d'élimination d'acides gras à chaîne courte ayant de 4 à 10 atomes de carbone par molécule présents dans une solution aqueuse de glycérine contenant des acides gras, dans lequel on envoie la solution aqueuse de glycérine d'une purification grossière, constituée sous la forme d'une centrifugeuse, à la cuve collectrice de pompage et ensuite à une unité de filtration de laquelle on soutire une solution de glycérine sous la forme d'un perméat et un rétentat contenant des acides gras à chaîne courte, le rétentat étant retourné à la cuve collectrice de pompage,
**caractérisé en ce que** l'on passe dans la cuve collectrice de pompage au-dessus de la limite de solubilité des acides gras à chaîne courte dans l'eau en sorte que des acides gras à chaîne courte se séparent de l'eau et s'accumulent sous la forme d'une couche continue sur la solution aqueuse de glycérine et **en ce que** l'on soutire les acides gras à chaîne courte de la cuve collectrice de pompage et on les élimine du procédé et **en ce que** l'on envoie le perméat sur un échangeur d'anions et on élimine ainsi les acides gras à chaîne courte restants de la solution de glycérine.

2. Procédé suivant la revendication 1, **caractérisé en ce que** l'unité de filtration est constituée sous la forme d'une microfiltration.

3. Procédé suivant la revendication 1, **caractérisé en ce que** l'unité de filtration est constituée sous la forme d'une nanofiltration.

4. Procédé suivant la revendication 1, **caractérisé en ce que** l'unité de filtration est constituée sous la forme d'une unité d'ultrafiltration.
